# EUROPEAN PATENT APPLICATION

(11) **EP 2 674 478 A1**
(43) Date of publication of application: **18.12.2013**
(21) Application number: 13170979.2
(22) Date of filing: 07.06.2013
(51) Int. Cl.: C12M 1/00, C12M 1/12, C12M 1/42, C12M 3/00

(54) **Culture device**

(30) Priority: 13.06.2012 TW 101121012
(71) Applicant: Chang, Sheng-Nan, 80457 Kaohsiung City (TW); Chang, Sheng-Chih, 80457 Kaohsiung City (TW)
(72) Inventor: Chang, Sheng-Nan, 80457 Kaohsiung City (TW); Chang, Sheng-Chih, 80457 Kaohsiung City (TW); Ay, Chyung, 80457 Kaohsiung City (TW); Chen, Cheng-Nan, 80457 Kaohsiung City (TW)
(74) Representative: Heine, Christian Klaus

(57) **Abstract**

A culture device (4) includes a deformable cell-culture unit (5) that includes a culture membrane (51) and a surrounding wall (52) extending upwardly from a periphery of the culture membrane (51), and a deformable engaging unit (6) that is connected to the surrounding wall (52) of the deformable cell-culture unit (5), that is adapted to engage a stretching device (7), and that has a hardness larger than that of the cell-culture unit (5).

## Description

This invention relates to a culture device, more particularly to a culture device adapted to engage a stretching device.

In a living subject, cells of the subject are usually in a dynamic state and are subjected to force stimulation. Such force stimulation resulting from, e.g., body movement (such as walking, running, etc.), blood-vessel pulsation, respiration,peristalsis,andfiltrationin urinarysystem would cause deformation of cells. The force stimulation not only influences morphology of the cells but also affects cell proliferation, cell differentiation, apoptosis, cell migration, remodeling of extracellular matrix and gene expression.

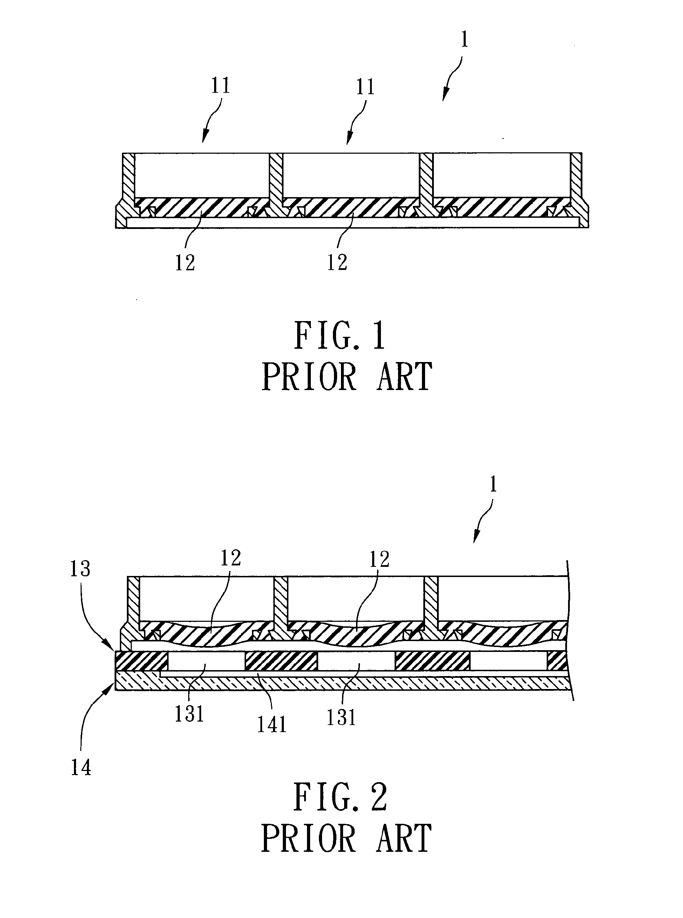

In order to provide a mimic physical stimulation for the cells, as shown in Figure 1, U.S. Patent No. 4789601 discloses a cell culture apparatus 1 including a plurality of wells 11, each of which contains an elastomeric membrane 12 for culturing cells thereon. When the cells are to be supplied with physical stimulation, as shown in Figure 2, a rubber gasket 13 formed with a plurality of apertures 131 is superposed on a plenum 14 formed with a plurality of vacuum channels 141. The vacuum channels 141 are in fluid communication with the apertures 131. The cell culture apparatus 1 is superposed on the rubber gasket 13, and vacuum equipment is used to pull the elastomeric membrane 12 downwardly by extracting air from the vacuum channels 141. When the air extraction is stopped, the elastomeric membranes 12 return to the original position shown in Figure 1. The deformation of the elastomeric membrane 12 would provide physical stimulation to the cells cultured on the elastomeric membrane 12.

Although the aforementioned equipment and operating method can provide physical stimulation for the cells, due to elastic fatigue, the elastomeric membranes 12 are likely to be permanently distorted even without air extraction. Therefore, the force stimulation and the subsequent observation of cell morphology are adversely influenced. Furthermore, the vacuum equipment would make loud noise and the deformation degree would be undesirably changed due to the compressible property of the gas.

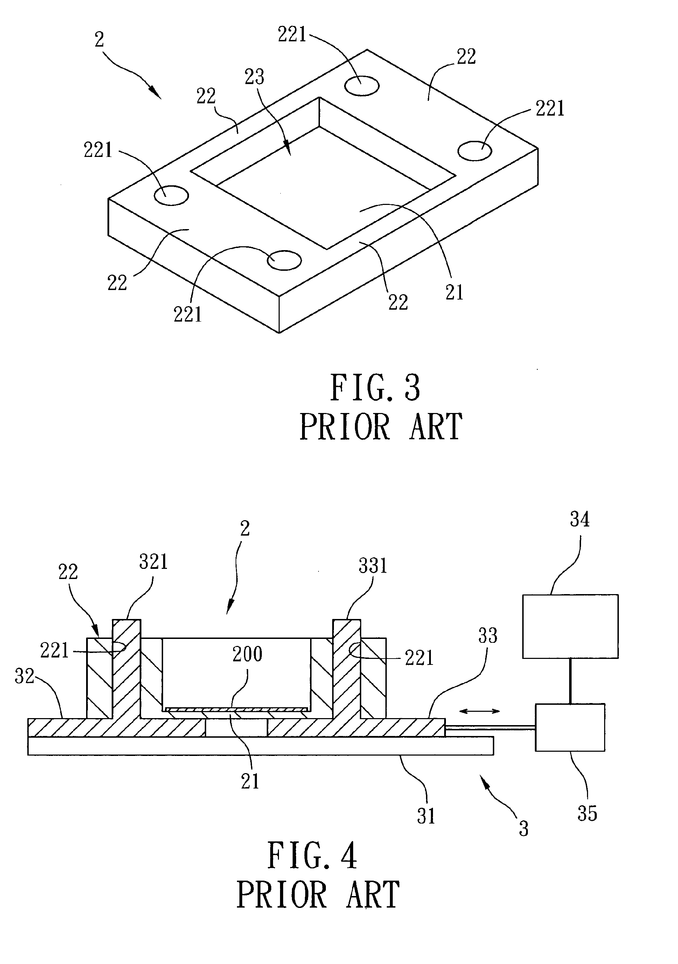

In order to solve the aforementionedproblems, as shown in Figure 3, European Patent Application Publication No. 1734110 A1 discloses a culture device 2 having elastic and deformable properties. The culture device 2 includes a bottom membrane 21 and side walls 22 upstanding from the entire periphery of the bottom membrane 21. The bottom membrane 21 and the side walls 22 cooperatively define a space 23 for culturing cells 200 (see Fig. 4). Engagement holes 221 are formed in opposing side walls 22 on a line extended from a periphery edge of the bottom membrane 21.

As shown in Figure 4, a stretching device 3 is used to engage the culture device 2 to provide stretching force to the culture device 2. The stretching device 3 includes a rail plate 31, a fixed plate 32 which is fixed to the rail plate 31, a movable plate 33 which is disposed slidably on the rail plate 31, and pins 321, 331 protruding from the fixed plate 32 and the movable plate 33, respectively. In use, the pins 321, 331 are inserted into the engagement holes 221 of the culture device 2. A step motor 35 controlled by a control device 34 is used to move the movable plate 33 away from the fixed plate 32. Thus, a force would be transmitted to the side wall 22 via the pins 331, and the bottom membrane 21 would be stretched, thereby providing physical stimulation to the cells 200 cultured on the bottom membrane 21.

Even though the aforesaid culture device 2 can solve the problems encountered in U.S. Patent No. 4789601, the force transmitted to the side walls 22 from the pins 331 would be gradually decreased from the pins 331. Therefore, the bottom membrane 21 may be pulled and deformed unevenly. In addition, the side walls 22 have the properties of elasticity and deformability that are liable to result in permanent distortion of the engagement holes 221 after long term use, thereby resulting in more uneven stretching force.

Moreover, if the hardness of the side walls 22 is designed to be identical to that of the bottom membrane 21, in order to satisfy deformation requirement of the bottom membrane 21, the side walls 22 may not be hard enough to stand against the stretching force. If the hardness of the culture device 2 is raised entirely, the bottom membrane 21 may be too hard to be deformed.

Therefore, the object of the present invention is to provide a culture device that can overcome the aforesaid drawbacks of the prior art.

According to a first aspect of this invention, there is provided a culture device adapted to engage a stretching device, which includes a deformable cell-culture unit that includes a culture membrane and a surrounding wall extending upwardly from a periphery of the culture membrane, and a deformable engaging unit that is connected to the surrounding wall of the deformable cell-culture unit. The engaging unit is adapted to engage the stretching device, and that has a hardness larger than that of the cell-culture unit.

Other features and advantages of the present invention will become apparent in the following detailed description of the preferred embodiments with reference to the accompanying drawing, of which:
Figure 1 is a cross-sectional view of a conventional cell culture apparatus disclosed in U.S. Pat. No. 4789601;
Figure 2 is a cross-sectional view showing deformation of elastomeric membranes of the cell culture apparatus of Figure 1, in which a rubber gasket and a plenum are used to perform such deformation;
Figure 3 is a perspective view of a conventional culture device disclosed in EP 1734110 A1;
Figure 4 is a partly cross-sectional view showing use of the culture device of Figure 3 with a stretching device;
Figure 5 is an exploded perspective view showing the first preferred embodiment of a culture device according to this invention;
Figure 6 is an assembled perspective view showing the first preferred embodiment;
Figure 7 is a side view showing use of the first preferred embodiment with a stretching device;
Figure 8 is a side view showing detailed structure of a connecting portion included in the first preferred embodiment;
Figure 9 is an exploded perspective view showing the second preferred embodiment of a culture device according to this invention; and
Figure 10 is an assembled perspective view showing the second preferred embodiment.

Before the present invention is described in greater detail with reference to the accompanying preferred embodiments, it should be noted herein that like elements are denoted by the same reference numerals throughout the disclosure.

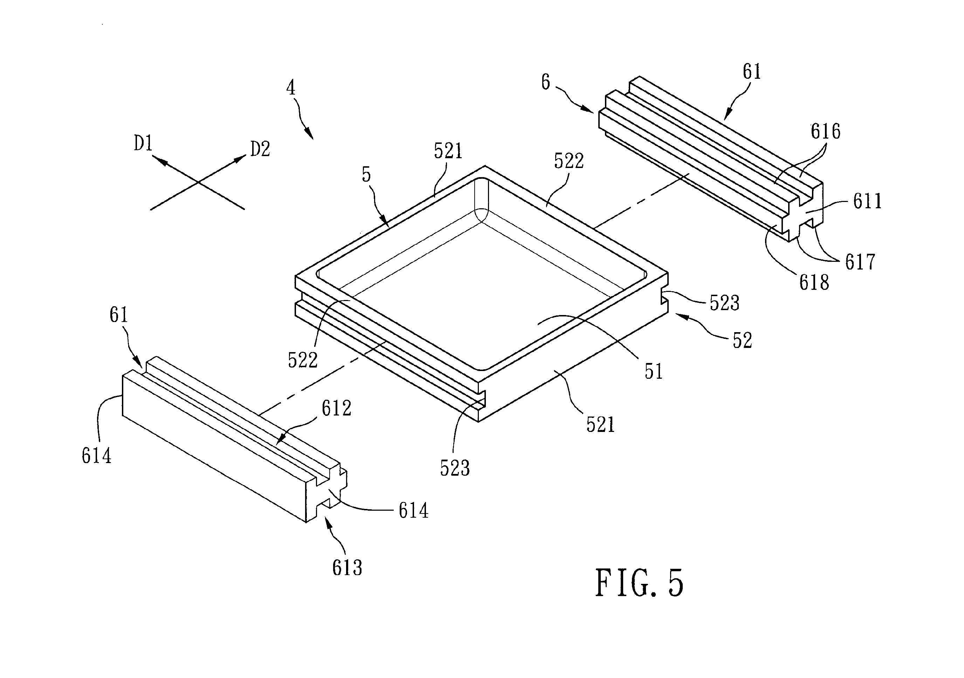

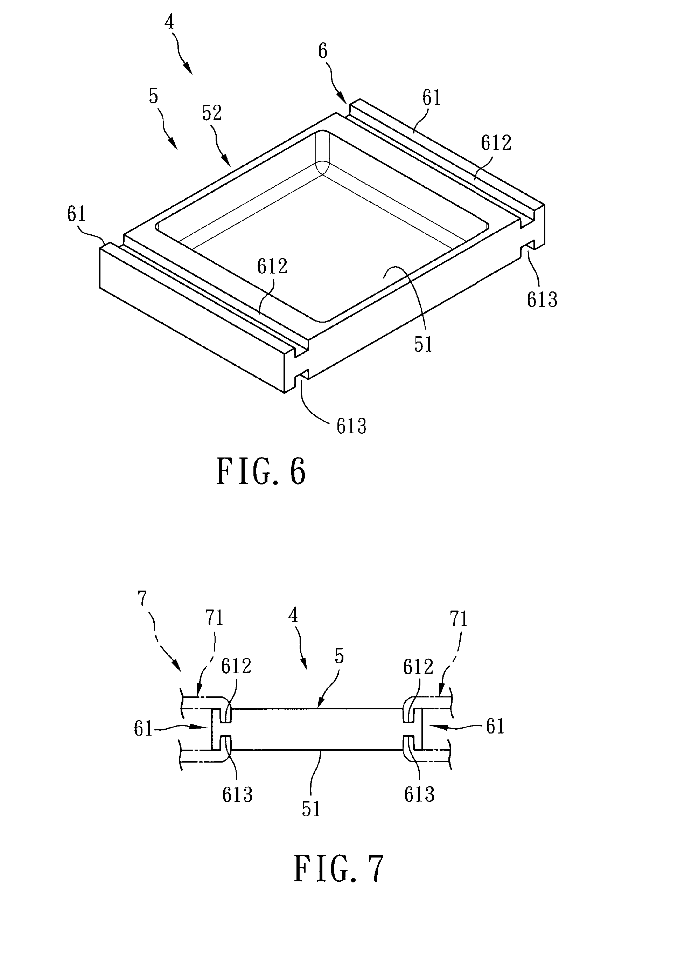

Referring to Figs. 5 and 6, the first preferred embodiment of a culture device 4 of the present invention includes a deformable cell-culture unit 5 and a deformable engaging unit 6. The engaging unit 6 is connected to the cell-culture unit 5 and has a hardness larger than that of the cell-culture unit 5. The cell-culture unit 5 is stretchable and the hardness thereof ranges between 10 Shore A and 59 Shore A. The engaging unit 6 is stretchable and the hardness thereof ranges between 60 Shore A and 90 Shore A. Preferably, the hardness of the cell-culture unit 5 is 30 Shore A, and the hardness of the engaging unit 6 is 70 Shore A.

In this embodiment, the cell-culture unit 5 includes a rectangular culture membrane 51 and a surrounding wall 52 which extends upwardly from a periphery of the culture membrane 51. The surrounding wall 52 is also in a shape of a rectangle and has two opposite first side walls 521 and two opposite second side walls 522 interconnecting the first side walls 521.

The engaging unit 6 is connected to the surrounding wall 52 of the cell-culture unit 5. To be specific, the engaging unit 6 includes two connecting portions 61 connected to the second side walls 522 of the surrounding wall 52, respectively.

Each of the connecting portions 61 has upper and lower surfaces and is formed with opposite upper and lower grooves 612, 613 that are indented inwardly from the upper and lower surfaces, respectively. Each of the connecting portions 61 further has opposing end surfaces 614 that interconnect the upper and lower surfaces and that are, in this embodiment, flush with the first side walls 521, respectively. The upper and lower grooves 612, 613 of each of the connecting portions 61 extend along a longitudinal direction (D1) of the second side walls 522 of the surrounding wall 52 and terminate at the end surfaces 614.

Each of the connecting portions 61 includes a body segment 611, two upper tooth segments 616 extending upwardly from the body segment 611 and cooperatively defining the upper groove 612 with the body segment 611, and two lower tooth segments 617 extending downwardly from the body segment 611 and cooperatively defining the lower groove 613 with the body segment 611.

Each of the second side walls 522 of the surrounding wall 52 in Figure 5 has an indent 523. Each of the connecting portions 61 further includes a protrusion 618 extending from the body segment 611 in a lateral direction (D2) perpendicular to the longitudinal direction (D1) and engaging the indent 523 of a respective one of the second side walls 522. Alternatively, the indent 523 can be formed in each of the connecting portions 61, and the protrusion 618 can be formed on each of the second side walls 522. The indent-protrusion structure would increase contact surface area, thereby improving connecting force between the protrusion 618 and the indent 523 and prolonging service life.

Each of the cell-culture unit 5 and the engaging unit 6 can be made from, e.g., silicone, polydimethylsiloxane (PDMS), ε-caprolactone (PCL), or poly(latide-co-glycolide acid) (PLGA). In this embodiment, the cell-culture unit 5 and the engaging unit 6 are made from silicone. The hardness of the cell-culture unit 5 and the engaging unit 6 can be controlled by process conditions or addition of a curing agent. The culture membrane 51 is preferably transparent and has a thickness suitable for observation under a microscope, e.g., 0.2 mm. Upon assembling, the protrusion 618 and the indent 523 align and engage with each other, followed by a thermocompression bonding so as to form the culture device 4 (see Figure 6). Alternatively, the culture device 4 can be made by injection molding.

It should be noted that the shape of the culture device 4 is not limited to the rectangular shape of this embodiment and can be other shapes suitable for this invention.

Figure 7 shows use of the culture device 4 with a stretching device 7. The stretching device 7 includes a clamping unit 71. In order to provide even forces to cells formed on the culture membrane 51, preferably, the clamping unit 71 has a length in the longitudinal direction (D1), that is substantially the same as that of the upper and lower grooves 612, 613 of each of the connecting portions 61. In use, the clamping unit 71 is retained in the upper and lower grooves 612, 613 of each of the connecting portions 61, and the stretching device 7 provides a force to the culture device 4 so that the cell-culture unit 5 and the connecting portions 61 are stretched so as to provide physical stimulation to the cells.

To be specific, when the culture device 4 has a rectangular shape, one of the connecting portions 61 can be fixed and the other one of the connecting portions 61 is pulled by the stretching device 7 so that the culture membrane 51 is stretched in the lateral direction (D2). Since the structure and the operation of the stretching device 7 are not the features of this invention, detailed descriptions thereof are omitted herein for the sake of brevity.

It should be noted that the shape of the grooves should not be limited in this preferred embodiment, and can be modified based on the configuration of the clamping unit 71 of the stretching device 7.

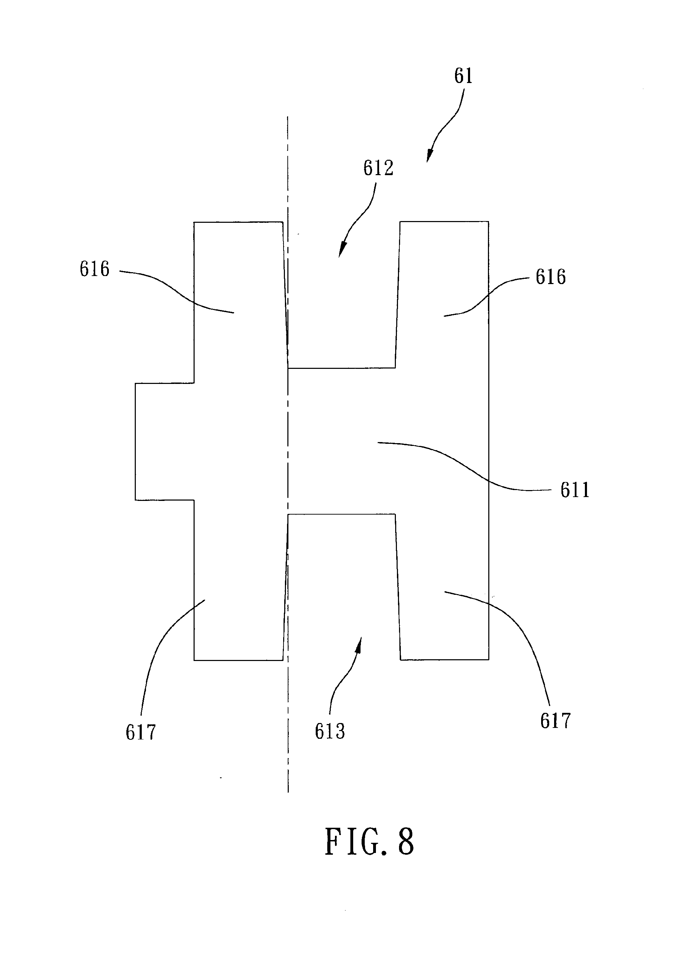

As shown in Figure 8, preferably, the upper and lower grooves 612, 613 of each of the connecting portions 61 are tapered inwardly from the upper and lower surfaces of each of the connecting portions 61, respectively. To be specific, the upper groove 612 of each of the connecting portions 61 has a width in the lateral direction (D2). The width is increased from the body segment 611 to a tip of each of the upper tooth segments 616. Similarly, a width of the lower groove 613 of each of the connecting portions 61 in the lateral direction (D2) is increased from the body segment 611 to a tip of each of the lower tooth segments 617. As such, the upper and lower grooves 612, 613 can easily and quickly engage the clamping unit 71.

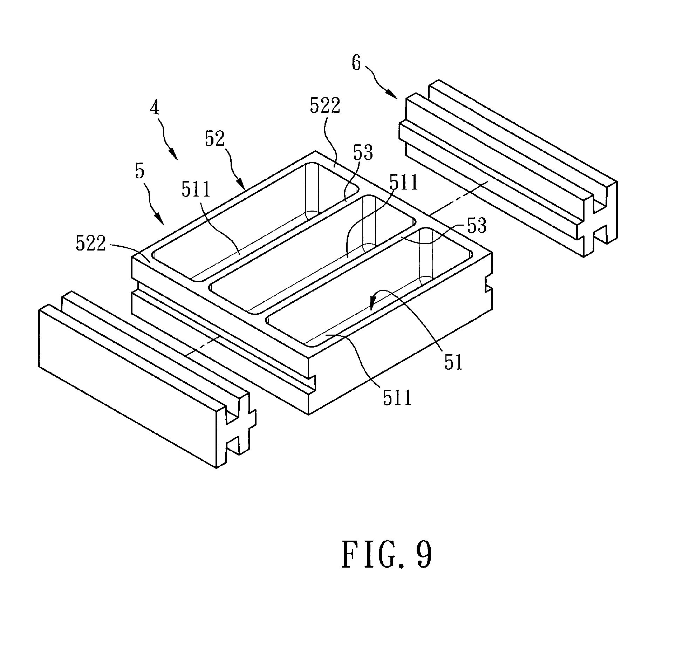

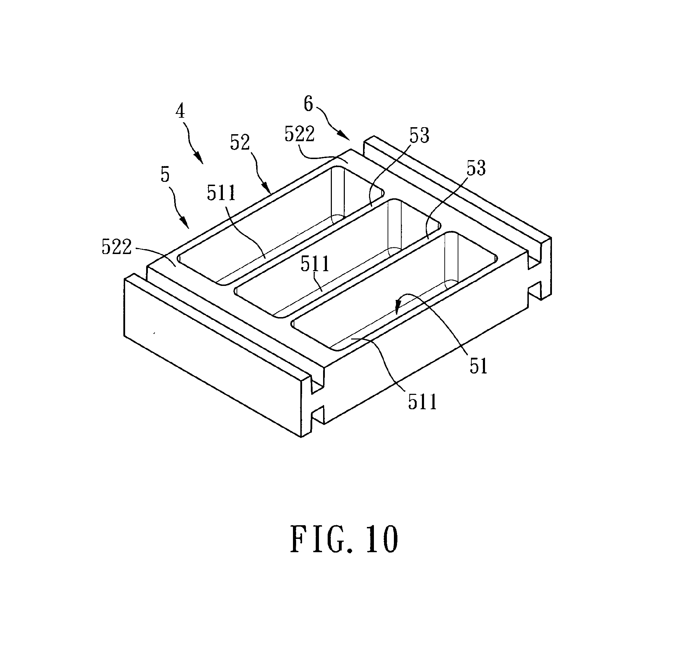

Figures 9 and 10 show the second preferred embodiment of the culture device 4 of the present invention. The second preferred embodiment is similar to the first preferred embodiment except that, in this embodiment, the cell-culture unit 5 further includes two partition walls 53 each of which interconnects the second side walls 522 of the surrounding wall 52 so as to divide the culture membrane 51 into three spaced-apart culturing regions 511. The number of the partition walls 53 could vary depending on actual requirements.

With the partition walls 53, the cells can be cultured and assessed in different experimental conditions under an identical stretching environment, thereby improving experimental convenience. Moreover, since the partition walls 53 are designed to extend in the lateral direction (D2) which is parallel to a stretching direction, the force applied to each of the culturing regions 511 would be substantially identical.

To sum up, since the hardness of the engaging unit 6 is larger than that of the cell-culture unit 5, the engaging units 6 can endure greater stretching force and provide desirable hardness and elasticity to the culture device 4. Moreover, since the upper and lower grooves 612, 613 are designed to extend along the longitudinal direction (D1) and to terminate at the end surfaces 614, when the same engage the aforesaid clamping unit 71, a relatively even stretching force can be applied to the culture device 4.

## Claims

1. A culture device (4) adapted to engage a stretching device (7), **characterized by**:
a deformable cell-culture unit (5) that includes a culturemembrane (51) and a surrounding wall (52) extending upwardly from a periphery of said culture membrane (51); and
a deformable engaging unit (6) that is connected to said surrounding wall (52) of said deformable cell-culture unit (5), that is adapted to engage the stretching device (7), and that has a hardness larger than that of said cell-culture unit (5).

2. The culture device (4) of claim 1, **characterized in that** said surrounding wall (52) has two opposite side walls (521, 522), said engaging unit (6) including two connecting portions (61) connected to said two opposite side walls (521, 522).

3. The culture device (4) of claim 2, **characterized in that** each of said connecting portions (61) has upper and lower surfaces and is formed with upper and lower grooves (612, 613) that are indented inwardly from said upper and lower surfaces, respectively, and that extend along a longitudinal direction (D1) of said side walls (521, 522) of said surrounding wall (52), the stretching device (7) including a clamping unit (71) that is retained in said upper and lower grooves (612, 613) of each of said connecting portions (61) and that has a length in said longitudinal direction (D1), the length of the clamping unit (71) being the same as that of said upper and lower grooves (612, 613) of each of said connecting portions (61).

4. The culture device (4) of claim 3, **characterized in that** said upper and lower grooves (612, 613) of each of said connecting portions (61) are tapered inwardly from said upper and lower surfaces of each of said connecting portions (61), respectively.

5. The culture device (4) of claim 3, **characterized in that** each of said connecting portions (61) includes a body segment (611), two upper tooth segments (616) extending upwardly from said body segment (611) and cooperatively defining said upper groove (612) with said body segment (611), and two lower tooth segments (617) extending downwardly from said body segment (611) and cooperatively defining said lower groove (613) with said body segment (611).

6. The culture device (4) of claim 5, **characterized in that** each of opposite side walls (521, 522) of said surrounding wall (52) has an indent (523), each of said connecting portions (61) further including a protrusion (618) engaging said indent (523) in a respective one of said side walls (521, 522).

7. The culture device (4) of claim 1, **characterized in that** said cell-culture unit (5) further includes at least one partition wall (53) interconnecting opposing portions of said surrounding wall (52) to divide said culture membrane (51) into at least two spaced-apart culturing regions (511).

8. The culture device (4) of claim 1, **characterized in that** each of said cell-culture unit (5) and said engaging unit (6) is made of a material selected from the group consisting of silicone, polydimethylsiloxane (PDMS), ε-caprolactone (PCL), poly (latide-co-glycolide acid) (PLGA), and combinations thereof.

9. The culture device (4) of claim 1, **characterized in that** said culture membrane (51) of said deformable cell-culture unit (5) is light-transmissible.

10. The culture device (4) of claim 1, **characterized in that** saiddeformable cell-cultureunit (5) has a hardness ranging between 10 Shore A and 59 Shore A, said deformable engaging unit (6) having a hardness ranging between 60 Shore A and 90 Shore A.

11. The culture device (4) of claim 10, **characterized in that** the hardness of said deformable cell-culture unit (5) is 30 Shore A, the hardness of said deformable engaging unit (6) being 70 Shore A.
